# EUROPÄISCHE PATENTANMELDUNG

(11) **EP 4 843 661 A1**
(43) Veröffentlichungstag der Anmeldung: **25.11.2026**
(21) Anmeldenummer: 25178429.4
(22) Anmeldetag: 23.05.2025
(51) Int. Cl.: B01J 8/00, B01J 8/06, C07C 1/04

(54) **FLÜSSIGKEITSGEKÜHLTER REAKTOR ZUR EXOTHERMEN KATALYTISCHEN UMWANDLUNG EINES REAKTIONSGASES UND VERFAHREN ZUR METHANOLSYNTHESE**

(71) Anmelder: L'AIR LIQUIDE, SOCIETE ANONYME POUR L'ETUDE ET L'EXPLOITATION DES PROCEDES GEORGES CLAUDE, 75007 Paris (FR)
(72) Erfinder: Oelmann, Tobias, 60439 Frankfurt am Main (DE); Strozyk, Michael, 60439 Frankfurt am Main (DE); Schmitt, Thorsten, 60439 Frankfurt am Main (DE)
(74) Vertreter: Air Liquide

(57) **Zusammenfassung**

Die Erfindung betrifft einen flüssigkeitsgekühlten Reaktor (1) zur exothermen katalytischen Umwandlung eines Reaktionsgases, insbesondere zur Synthese von Methanol, umfassend, einen Reaktorbehälter mit einem Mantelraum (2), in dem vertikal angeordnete Katalysatorrohren (6) mit einer Katalysatorschüttung (8) befüllt sind, und eine Flüssigkeitskühlung (9), die dazu eingerichtet ist, dass die Katalysatorrohre (6) von einer Kühlflüssigkeit umströmt werden, um die bei der exothermen Reaktion entstehende Wärme abzuführen. In wenigstens einem der Katalysatorrohre (6) ist eine Temperaturmesseinrichtung (10) angeordnet, um die Temperatur in dem Katalysatorrohr (6) an mehreren Stellen entlang der Rohrachse (7) zu messen. Eine Halteanordnung fixiert die axiale Position der Temperaturmesseinrichtung (10) in dem Katalysatorrohr (6) und umfasst mehrere eines Abstandshalters (11), die in dem Katalysatorrohr (6) entlang der Rohrachse (7) verteilt sind und die dazu eingerichtet sind, die Temperaturmesseinrichtung (10) in einem definieren Abstand zur Innenwand des Katalysatorrohres (6) zu positionieren.

## Beschreibung

### TECHNISCHES GEBIET

Die Erfindung betrifft einen flüssigkeitsgekühlten Reaktor zur exothermen katalytischen Umwandlung eines Reaktionsgases, insbesondere zur Synthese von Methanol. Die Erfindung betrifft ferner ein Verfahren zur Methanolsynthese.

### HINTERGRUND DER ERFINDUNG

In der Industrie wird Methanol durch die heterogen-katalytische Umsetzung von Synthesegas hergestellt. Synthesegas umfasst verschiedene Gasgemische, die insbesondere Wasserstoff und Kohlenoxide enthalten. Die hierfür eingesetzten Reaktoren sind typischerweise als stehende Festrohrwärmetauscher ausgeführt. Aufgrund der exothermen Reaktion werden fluidgekühlte bzw. flüssigkeitsgekühlte Reaktoren eingesetzt, um eine Überhitzung des Katalysators und der Anlagentechnik zu vermeiden. Bei fluidgekühlten Reaktoren befindet sich der Katalysator innerhalb vertikaler Rohre, die von einer in der Regel wasserhaltigen Kühlflüssigkeit auf der Mantelseite umströmt werden. Die Rohre werden über obere und untere Rohrplatten mechanisch fixiert und abgestützt. Die Wärmeabfuhr erfolgt über das Kühlmedium, wobei Dampf entstehen kann, der zusammen mit der Flüssigkeit als Dampf-Flüssigkeits-Gemisch an den Rohren aufsteigt.

Durch die Weiterentwicklung von Katalysatoren konnte die Produktivität pro Reaktorvolumen deutlich gesteigert werden, sodass heute kompaktere Reaktoren mit höherer Effizienz eingesetzt werden. Gleichzeitig gewinnt die Temperaturüberwachung innerhalb des Reaktors an Bedeutung, da Katalysatoren im Betrieb altern und sich dadurch die Temperaturprofile verändern. Eine präzisere Überwachung kann eine bessere Steuerung der Temperaturentwicklung, eine gezielte Anpassung des Kreislaufverhältnisses sowie eine effizientere Planung des Katalysatoraustauschs ermöglichen.

Aus dem Stand der Technik ist bekannt, flüssigkeitsgekühlte Reaktoren durch simulative Bilanzierung zu überwachen. Dabei werden die Ein- und Austrittsströme des Reaktors sowie die erzeugte Dampfproduktion bilanziert. Ein wesentlicher Nachteil dieser Methode ist, dass dabei kein aussagekräftiges Temperaturprofil erstellt werden kann, weder innerhalb der Katalysatorrohre noch innerhalb des Reaktors. Ein Temperaturprofil beschreibt die Temperaturverteilung entlang der Katalysatorrohre oder im gesamten Reaktorraum. Ohne Temperaturprofil bleiben lokale Temperaturspitzen oder Kaltzonen unentdeckt. Solche Abweichungen können durch Katalysatorvergiftungen oder eine ungleichmäßige Durchströmung verursacht werden und beeinträchtigen die Reaktorleistung. Da sich der Alterungsfortschritt des Katalysators nicht direkt erkennen lässt, ist ein ungewöhnlicher Verschleiß schwer zu detektieren. Dadurch kann der optimale Zeitpunkt für den Katalysatoraustausch nicht frühzeitig bestimmt werden, was zu unerwarteten Leistungsverlusten oder ungeplanten Stillständen führen kann.

Es ist Aufgabe der Erfindung, die mit Bezug auf den Stand der Technik geschilderten Probleme zumindest teilweise zu lösen.

### ZUSAMMENFASSUNG DER ERFINDUNG

Die der Erfindung zugrundeliegende Aufgabe wird gelöst durch die Merkmale der unabhängigen Patentansprüche. Weitere vorteilhafte Ausgestaltungen der Erfindung sind in den abhängigen Ansprüchen und der Beschreibung angegeben. Die in den Ansprüchen und in der Beschreibung dargestellten Merkmale sind in beliebiger, technologisch sinnvoller Weise miteinander kombinierbar.

In einem ersten Aspekt der Erfindung wird ein flüssigkeitsgekühlter Reaktor zur exothermen katalytischen Umwandlung eines Reaktionsgases, insbesondere zur Synthese von Methanol, vorgestellt, umfassend einen Reaktorbehälter mit einem Mantelraum, eine Vielzahl von in dem Mantelraum vertikal angeordneten Katalysatorrohren, die sich entlang einer jeweiligen Rohrachse erstrecken und die mit einer Katalysatorschüttung befüllt sind, und eine Flüssigkeitskühlung, die dazu eingerichtet ist, dass die Katalysatorrohre von einer Kühlflüssigkeit umströmt werden, um die bei der exothermen Reaktion entstehende Wärme abzuführen. In wenigstens einem der Katalysatorrohre ist eine Temperaturmesseinrichtung angeordnet. Die Temperaturmesseinrichtung umfasst wenigstens einen Temperaturmessfühler und ist dazu angepasst, die Temperatur in dem Katalysatorrohr an mehreren Stellen entlang der Rohrachse zu messen. Eine Halteanordnung ist dazu eingerichtet, die axiale Position der Temperaturmesseinrichtung in dem Katalysatorrohr zu fixieren, wobei die Halteanordnung mehrere eines Abstandshalters umfasst. Die Abstandshalter sind in dem Katalysatorrohr entlang der Rohrachse verteilt und dazu eingerichtet, die Temperaturmesseinrichtung in einem definieren Abstand zur Innenwand des Katalysatorrohres zu positionieren.

Erfindungsgemäß wird unter einem Reaktor eine Vorrichtung verstanden, welche zur Durchführung einer chemischen Reaktion eingerichtet ist. Ein flüssigkeitsgekühlter Reaktor für Syntheseprozesse ist Reaktor, der mit einem flüssigen Kühlmedium arbeitet. Der beschriebene Reaktor ist vorzugsweise zur Methanolsynthese eingerichtet, kann jedoch für eine Vielzahl denkbarer chemischer Reaktionen genutzt werden, in welchen ein Reaktionsgas mittels eines Katalysators unter indirekter Kühlung mittels eines flüssigen Kühlmediums umgesetzt wird. Das Kühlsystem dient dazu, die Temperatur im Reaktor während chemischer Prozesse zu regulieren, Überhitzung zu vermeiden und eine optimale Reaktionsführung zu gewährleisten.

Nach einer bevorzugten Ausführungsform der Erfindung ist die Kühlflüssigkeit ein wasserhaltiges Kühlmedium und der flüssigkeitsgekühlte Reaktor ist ein wassergekühlter Reaktor. Das bedeutet nicht, dass die Kühlung des Reaktors zwangsläufig mit reinem Wasser erfolgen muss. Es kann eine Flüssigkeit verwendet werden, die Wasser enthält, wie etwa Wasser-Glykol-Gemische oder andere wasserbasierte Kühlflüssigkeiten. Der Begriff "wassergekühlter Reaktor" wird aus Vereinfachungsgründen verwendet, auch wenn das Kühlmedium nicht ausschließlich Wasser ist.

Der Reaktor umfasst einen Reaktorbehälter. Der Reaktorbehälter ist vorzugsweise als ein Druckbehälter ausgebildet. Der Reaktorbehälter umfasst einen vorzugsweise zylindrischen Reaktormantel und stellt einen Mantelraum bereit. Der Reaktorbehälter umfasst einen Reaktionsgaseinlass, um das Reaktionsgas in den Mantelraum des Reaktorbehälters einzuleiten. Der Mantelraum ist unterteilt. Obere und untere Rohrplatten, zwischen denen sich die Katalysatorrohre erstrecken, leiten das Reaktionsgas durch die Katalysatorrohre. Zwischen den Rohrplatten ist das Kühlfluid der Flüssigkeitskühlung angeordnet und umströmt die Katalysatorrohre. Das Reaktionsgas wird chemisch umgesetzt, während es durch die Katalysatorschüttungen in den Katalysatorrohren strömt, in der Regel nach unten. Über einen Reaktionsgasauslass kann das zumindest teilweise umgesetzte Reaktionsgas aus dem Reaktorbehälter ausgelassen werden. Aufgrund der in den Katalysatorrohren ablaufenden chemischen Reaktionen hat das Reaktionsgas am Reaktionsgasauslass im Allgemeinen eine andere chemische Zusammensetzung als am Reaktionsgaseinlass.

Die Temperaturmesseinrichtung erstreckt sich innerhalb des Katalysatorrohres entlang der Rohrachse und umfasst einen oder mehrere Temperaturmessfühler, um die Temperatur in dem Katalysatorrohr an mehreren Stellen entlang der Rohrachse zu messen.

Das schließt ein, dass die Temperaturmesseinrichtung durch einen oder mehrere Temperaturmessfühler bereitgestellt wird. Als geeignete Messfühler kommen unter anderem Mehrpunkt-Temperaturmessfühler wie Mehrpunkt-Thermoelemente und Mehrpunkt-Widerstandsthermometer in Betracht. Mit diesen können in vorteilhafter Weise mittels eines einzelnen Messfühlers mehrere Messstellen in übereinander liegenden Ebenen bereitgestellt werden. Einzelne Messfühler können aneinandergereiht werden. Bevorzugt ist der Temperaturmessfühler elastisch ausgeführt, was die Montage vereinfachen kann.

Durch die Anordnung von Temperaturmesspunkten entlang der Rohrachse kann ein präzises Temperaturprofil während des Betriebs des Reaktors erstellt werden. Messungen können zudem direkt in der Katalysatorschüttung vorgenommen werden. Die Abstandshalter gewährleisten, dass die Temperaturmesseinrichtung bzw. der Temperaturmessfühler an den Messpunkten von der Innenwand des von außen gekühlten Katalysatorrohres abgesetzt ist, wodurch eine Verfälschung der Messwerte vermieden wird. Die Abstandshalter sind so konzipiert, dass die Temperaturmesseinrichtung bzw. der Temperaturmessfühler an den Messpunkten optimal in der Strömung des Reaktionsgases positioniert ist. Bevorzugt sind die Abstandshalter dazu eingerichtet, die Temperaturmesseinrichtung bzw. den einen oder die mehreren Temperaturmessfühler zentral im Katalysatorrohr zu positionieren.

Nach einer weiteren Ausführungsform der Erfindung ist vorgesehen, dass der Abstandshalter einen zumindest abschnittsweise zylindrischen Grundkörper umfasst, der an der Innenwand des Katalysatorrohres positioniert ist und von dem sich ein oder mehrere Distanzhalter zumindest teilweise radial nach innen, also in Richtung Mittelachse (Zylinderachse) des Grundkörpers, erstrecken und eine Führung für die Temperaturmesseinrichtung bereitstellen. Die Mittelachse des Grundköpers ist entlang der Rohrachse ausgerichtet, bevorzugt konzentrisch, d.h. der Abstandshalter ist bevorzugt mittig im dem Katalysatorrohr aufgenommen. Der zylindrische Grundkörper ist im Wesentlichen hohl, also eine Art Hülse, mit offenen Enden, sodass das Reaktionsgas durch den Abstandshalter strömen kann, aber auch die Katalysatorschüttung nicht wesentlich durch den Abstandshalter verdrängt wird. Über den zylindrischen Grundkörper kann sich der Abstandshalter gegenüber der Innenwand des Katalysatorrohrs effizient abstützen. Der Distanzhalter, der als eine Art Arm ausgestaltet sein kann, erstreckt sich vom Mantel des zylindrischen Grundkörpers in Richtung der Mittelachse und damit weg von der Innenwand des Katalysatorrohres hin zur Rohrachse. Der Distanzhalter kann ein freies, distales Ende aufweisen, an dem die Führung für die Temperaturmesseinrichtung bzw. den Temperaturmessfühler bereitgestellt ist, um diesen in einem Abstand zur Innenwand des Katalysatorrohres zu positionieren. Alternativ kann der Distanzhalter auch als eine Art Steg ausgebildet sein, der sich von einer Seite des Grundkörpers zur anderen Seite erstreckt.

Nach einer weiteren Ausführungsform der Erfindung ist vorgesehen, dass die Abstandshalter an ihrem entlang der Mittelachse gelegenen ersten und/oder zweiten Ende, also an den Stirnflächen, eine Schräge aufweisen, bzw. konisch geformt sind, um das Hindurchführen bzw. Gleiten durch das Katalysatorrohr zu erleichtern.

Nach einer weiteren Ausführungsform der Erfindung ist vorgesehen, dass der Abstandshalter mehrere Distanzhalter umfasst, die entlang der Rohrachse in einem Abstand zueinander verteilt sind. Bevorzugt sind die Distanzhalter in Achsenrichtung des Katalysatorrohres gesehen in einem Winkel zueinander versetzt; weiter vorzugsweise gleichmäßig um die Rohrachse verteilt. Bezogen auf die vertikal ausgerichtete Rohrachse sind die Distanzhalter auf unterschiedlichen Ebenen angeordnet. Weiter sind benachbarte Distanzhalter eines Abstandshalters bevorzugt gleichmäßig voneinander beabstandet. Besonders bevorzugt beträgt der vertikale Abstand zwischen benachbarten Distanzhaltern von 10 mm bis 15 mm. Die Orientierungen der Distanzhalter eines Abstandshalters können unterschiedlich sein, bevorzugt beträgt dabei der Winkelabstand der Distanzhalter in einem Abstandshalter in Rohrachsenrichtung gesehen: Winkelabstand = (360°/n), wobei n= Anzahl der Distanzhalter. Besonders bevorzugt weist der Abstandshalter drei in Rohrachsenrichtung bzw. Mittelachsenrichtung voneinander beabstandete Distanzhalter mit einem Winkelabstand von 120° auf.

Nach einer weiteren Ausführungsform der Erfindung ist vorgesehen, dass der oder die Distanzhalter des Abstandshalters in wenigstens einer Richtung entlang der Rohrachse (also entlang der Rohrachse gesehen) eine schräge oder abgerundete Fläche aufweist; bevorzugt eine Schräge in einem Winkel von 40° bis 50°, besonders bevorzugt 45°, zur Rohrachse. Die axiale Stirnfläche des Distanzhalters steht dadurch nicht senkrecht zur Gasströmung, sondern schräg. Dies verbessert nicht nur die Gasströmung durch den Abstandshalter, sondern erlaubt es dem Katalysator, durch den Abstandshalter mit geringem Blockaderisiko zu fallen, beispielweise beim Befüllen und Entleeren. Zudem erleichtert diese Konstruktion relative Bewegungen zwischen Katalysator und Abstandshalter.

Nach einer weiteren Ausführungsform der Erfindung ist vorgesehen, dass der zylindrische Grundkörper ein oder mehrere Aussparungen in einer umlaufenden Seitenwand aufweist. Aussparungen sind insbesondere Fenster und Öffnungen in der umlaufenden Seitenwand, d.h. der zylindrische Grundkörper ist nicht durchgängig zylindrisch. Die Aussparungen sind bevorzugt abschnittsweise und/oder zwischen benachbarten Distanzhaltern ausgebildet. Die Aussparungen sind möglichst derart ausgebildet, dass ihre Ausdehnung in Umfangsrichtung größer ist als die vorhandene Seitenwandfläche in dem betreffenden Abschnitt. Besonders bevorzugt weist der Abstandshalter nur im Bereich der Distanzhalter eine vollumfängliche Seitenwand auf, während der Bereich zwischen benachbarten Distanzhaltern überwiegend ausgespart ist. Das bedeutet, dass der zylindrische Grundkörper mehrere zylindrische Abschnitte umfasst, die durch teilweise offene Abschnitte miteinander verbunden sind. Abschnitte des Abstandshalters mit einem Distanzhalter können durch sich in Achsenrichtung erstreckende Stege miteinander verbunden sein, wobei die Stege vorzugsweise gleichmäßig um die Mittelachse herum verteilt sind. Die Aussparungen des Abstandshalters vermindern den Flächenkontakt mit der Innenwand des Katalysatorrohrs. So wird die Wärmeübertragung zwischen der Innenwand des Rohrs und dem Temperaturmessfühler noch effizienter gemindert.

Nach einer weiteren Ausführungsform der Erfindung ist vorgesehen, dass der Abstandshalter ein oder mehrere Randabweiser umfasst, wobei der Randabweiser dazu angepasst ist, einen direkten Kontakt zwischen dem Außenumfang des Abstandshalters und der Innenwand des Katalysatorrohres zu verhindern. Bevorzugt sind mehrere Randabweiser entlang der Mittelachse des zylindrischen Grundkörpers vorgesehen. Die Randabweiser können eine Vielzahl von insbesondere federnd gelagerten Abweiserarmen aufweisen, die sich in Richtung der Innenwand des Katalysatorrohrs erstrecken. Diese Randabweiser können dazu beitragen, die Reibung zur Innenwand des Katalysatorrohrs zu verringern, wodurch sie insbesondere das Einbringen des Abstandshalters in das Katalysatorrohr erleichtern. Die Randabweiser sind bevorzugt dazu eingerichtet, den Abstandshalter im Rohr zu zentrieren. Dies kann insbesondere dadurch erzielt werden, dass sich der Randabweiser um die Mittelachse herum erstreckt und mehrere Randabweiser beabstandet voneinander entlang der Mittelachse des Abstandshalters vorgesehen sind.

Nach einer weiteren Ausführungsform der Erfindung ist vorgesehen, dass die Halteanordnung Arretierungen umfasst, die an einer oberen und unteren Rohrplatte des Reaktors, zwischen denen sich die Katalysatorrohre erstrecken, festgelegt sind. Die Arretierungen sind derart mit der Temperaturmesseinrichtung verbunden, dass eine axiale Verschiebung der Temperaturmesseinrichtung bzw. der Temperaturmesspunkte verhindert wird.

Nach einer weiteren Ausführungsform der Erfindung ist vorgesehen, dass die Temperaturmesseinrichtung in einer Hülse aufgenommen ist. Die Hülse erstreckt sich in Rohrachsenrichtung und kann einen zusätzlichen Schutz für die Temperaturmesseinrichtung bereitstellen. Die Hülse erstreckt sich bevorzugt wenigstens vom obersten zum untersten Abstandshalter. Bevorzugt ist die Hülse an den Arretierungen festgelegt. Die Hülse ist an den Führungen der Distanzhalter angeordnet. Bevorzugt ist die Führung derart, dass eine axiale Bewegung des Temperaturmessfühlers bzw. der Hülse unterbunden aber eine rotatorische Bewegung möglich ist. An einem Ende der Hülse kann der Temperaturmessfühler festgelegt sein. Am anderen Ende der Hülse kann der Temperaturmessfühler aus der Hülse austreten. Es hat sich als vorteilhaft herausgestellt, wenn beide Enden der Hülse verschlossen sind, entweder temporär oder permanent.

Nach einer weiteren Ausführungsform der Erfindung ist vorgesehen, dass die Führung der Distanzhalter eine Buchse umfasst, in der die Hülse und/oder die Temperaturmesseinrichtung angeordnet ist.

Nach einer weiteren Ausführungsform der Erfindung ist vorgesehen, dass die Hülse mehrere Hülsensegmente umfasst, die axial miteinander koppelbar sind. Bevorzugt sind zumindest einige der Hülsensegmente jeweils an einem Abstandhalter angebracht, sodass das Hülsensegment zusammen mit dem Abstandshalter als Baugruppe mit wenigstens einem weiteren Hülsensegment koppelbar ist. Die Hülsensegmente können nacheinander in das Rohr eingeführt werden, was die Installation in engen oder schwer zugänglichen Bereichen vereinfacht, beispielsweise durch Inspektionsöffnungen. Dies ermöglicht eine einfachere Handhabung als bei der Verwendung einer durchgängigen Hülse. Statt einer einzigen langen Hülse werden mehrere Hülsensegmente schrittweise zusammengefügt, was den Platzbedarf bei der Montage minimiert. Die Hülsensegmente können miteinander gekoppelt werden, beispielsweise durch Verschrauben oder andere bevorzugt lösbare Verbindungen. Zumindest einige der Hülsensegmente sind als Baugruppe mit einem Abstandshalter ausgestattet und an diesem fixiert, was die Montage der Temperaturmesseinrichtung zusätzlich erleichtert.

Wie erläutert, kann eine durchgängige Hülse oder eine aus Hülsensegmenten zusammengesetzte Hülse verwendet werden. Die segmentierte Hülse hat insbesondere den Vorteil einer vereinfachten Installation. Ein erstes Segment kann mit einem Abstandshalter in das Katalysatorrohr so weit eingeführt werden, dass das Ende noch mit dem nächsten Segment verbunden werden kann. Das Einführen und Verbinden wiederholt sich dann so lange, bis das zuerst eingeführte Segment auf der anderen Seite des Katalysatorrohrs an der anderen Rohrplatte austritt und dort mit der Arretierung verbunden werden kann. Das letzte Segment kann dann mit der anderen Arretierung verbunden werden. Diese zweite Arretierung auf der Einführseite fixiert dann das Hülsensegmentsystem. Das Einführen kann durch Einschieben oder durch eine Kombination von Einschieben und unterstütztem Ziehen erfolgen. Ein unterstütztes Ziehen kann durch ein zuvor durchgeführtes Zugsystem erfolgen, das beispielsweise ein Seil, ein Draht oder eine Stange sein kann. Das Einziehen kann sowohl in horizontaler als auch in vertikaler Lage der Rohre erfolgen. Ein Einbau einer segmentieren Hülse kann sowohl in einen neuen Reaktor als auch in einen schon betriebenen Reaktor, zum Beispiel während eines Turnarounds, einem Wartungs- und Inspektionsprozess, durchgeführt werden.

Nach einer weiteren Ausführungsform der Erfindung ist vorgesehen, dass zumindest zwischen einigen benachbarten Hülsensegmenten ein Element, z.B. eine Scheibe, aus wärmeisolierendem Material vorgesehen ist. Das Material hat eine geringere Wärmeleitfähigkeit als das Material der Hülsensegmente.

Nach einer weiteren Ausführungsform der Erfindung ist vorgesehen, dass die Buchse aus wärmeisolierendem Material bereitgestellt ist, d.h., das Material der Buchse hat eine geringere Wärmeleitfähigkeit als das Material der Hülse und/oder des Distanzhalters.

In einem weiteren Aspekt betrifft die Erfindung ein Verfahren zur Methanolsynthese unter Verwendung eines Reaktors, wie er hier beschrieben ist. Bei dem Verfahren wird ein Synthesegas durch die Katalysatorrohre geleitet und zumindest teilweise umgesetzt. Eine Temperaturmessung wird an mehreren Stellen entlang der Rohrachse eines oder mehrerer Katalysatorrohre mittels einer in dem jeweiligen Katalysatorrohr angeordneten Temperaturmesseinrichtung vorgenommen. Bevorzugt wird auf Grundlage einer vorgenommenen Temperaturmessung ein Temperaturprofil erstellt.

### FIGURENBESCHREIBUNG

Die Erfindung wird nachfolgend anhand von Figuren näher erläutert. Die Figuren zeigen bevorzugte Ausführungsbeispiele, auf die die Erfindung jedoch nicht begrenzt ist. Die Figuren und die darin dargestellten Größenverhältnisse sind nur schematisch. Es zeigen:
- FIG. 1: schematisch die Anordnung einer Temperaturmesseinrichtung in einem Katalysatorrohr eines flüssigkeitskühlten Reaktors nach einer ersten Ausführungsform der Erfindung,
- FIG. 2: eine perspektivische schematische Darstellung eines erfindungsgemäßen Abstandshalters;
- FIG. 3: eine weitere Darstellung des Abstandshalters aus FIG. 2;
- FIG. 4: Details eines Abstandshalters nach einer weiteren Ausführungsform der Erfindung;
- FIG. 5: schematisch die Anordnung einer Temperaturmesseinrichtung in einem Katalysatorrohr eines flüssigkeitsgekühlten Reaktors nach einer weiteren Ausführungsform der Erfindung; und
- FIG. 6: eine perspektivische schematische Darstellung eines Abstandshalters nach einer weiteren Ausführungsform der Erfindung.

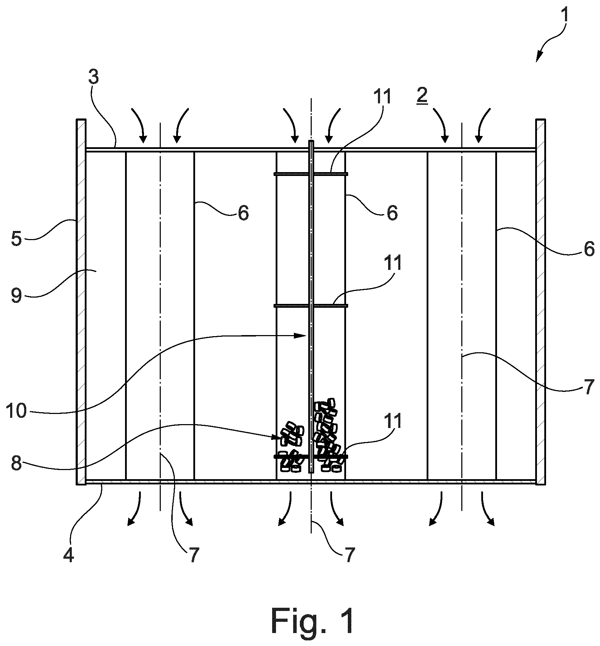

**FIG. 1** ist eine schematische Darstellung eines Abschnitts eines flüssigkeitsgekühlten Reaktors 1 zur exothermen katalytischen Umwandlung eines Reaktionsgases in der Schnittansicht. Der Reaktor 1 ist als wassergekühlter Reaktor bereitgestellt und umfasst einen Mantelraum 2, in dem eine obere Rohrplatte 3 und eine untere Rohrplatte 4 angeordnet sind, die sich zwischen einer umlaufenden Reaktorwand 5 erstrecken. Eine Vielzahl von vertikal ausgerichteten Katalysatorrohren 6 erstrecken sich entlang ihrer jeweiligen Rohrachse 7 zwischen den Rohrplatten 3 und 4, wobei die offenen Enden der Katalysatorrohre 6 mit dem Raum oberhalb der oberen Rohrplatte und dem Raum unterhalb der unteren Rohrplatte 4 in Fluidverbindung stehen, sodass in den oberen Mantelraum 2 eingeleitetes Reaktionsgas durch die Katalysatorrohre 6 nach unten strömen kann (angedeutet durch Pfeile). Dabei strömt das Reaktionsgas durch eine in den Katalysatorrohren 6 angeordnete Katalysatorschüttung 8. Zwischen den Rohrplatten 3 und 4 werden die Katalysatorrohre 6 von einer wasserhaltigen Kühlflüssigkeit einer Flüssigkeitskühlung 9 umströmt.

In einem der Katalysatorrohre 6 ist eine Temperaturmesseinrichtung 10 angeordnet, die sich zentral durch das Katalysatorrohr 6 erstreckt. Die Temperaturmesseinrichtung 10 umfasst einen Mehrpunkt-Temperaturfühler, der an unterschiedlichen Stellen entlang der Rohrachse 7, also auf unterschiedlichen Höhen, Temperaturmessungen vornimmt. Um die zentrale Position der Temperaturmesseinrichtung 10 in dem Katalysatorrohr 6 sicherzustellen, sind entlang der Rohrachse 7 mehrere Abstandshalter 11 vorgesehen, die Teil einer Halteanordnung sind und die den Mehrpunkt-Temperaturfühler in einem definierten Abstand zur Innenwand des Katalysatorrohres 6 positionieren.

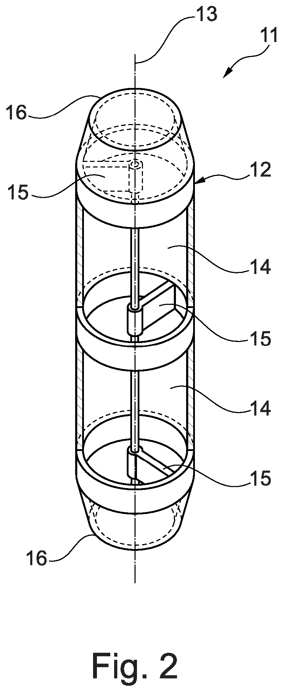

**FIG. 2** ist eine Darstellung eines Abstandshalters 11, wie er in einem Reaktor nach FIG. 1 verwendet werden kann. Der Abstandshalter 11 umfasst einen zylindrischen Grundkörper 12, der sich entlang einer Zylinderachse, die die Mittelachse 13 darstellt, erstreckt. Die vorderen und hinteren Enden des zylindrischen Grundkörpers 12 sind offen. Der zylindrische Grundkörper 12 ist nicht durchgängig zylindrisch, sondern weist Aussparungen 14 in der Zylinderwand bzw. der umlaufenden Seitenwand derart auf, dass drei vollumfänglich zylindrische und voneinander beabstandete Abschnitte bereitgestellt sind. In diesen Abschnitten ist jeweils ein Distanzhalter 15 angeordnet. Der Distanzhalter 15 erstreckt sich von der umlaufenden Seitenwand des zylindrischen Grundkörpers 12 radial nach innen zur Mittelachse 13 hin. Das distale Ende der Distanzhalter 15 weist eine Führung für die Temperaturmesseinrichtung auf. Die Führung liegt auf der Mittelachse 13.

Der Abstandshalter 11 wird mit einem axialen Ende voran in das Katalysatorrohr eingeführt. Das vordere und hintere Ende des Abstandshalters 11 weisen eine Konusform 16 auf, um das Einführen und die Positionierung des Abstandshalters 11 im Katalysatorrohr zu erleichtern.

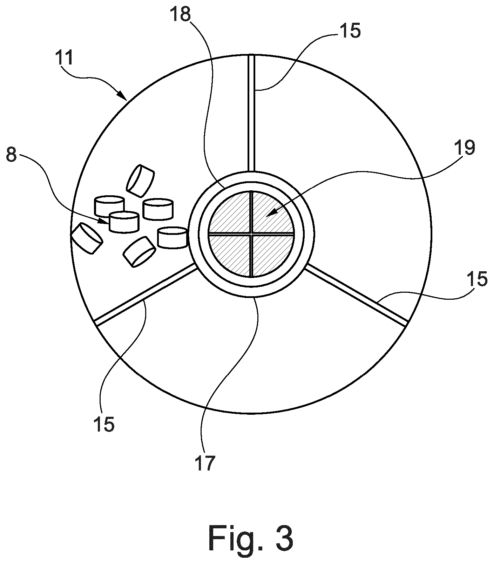

Die Distanzhalter 15 sind entlang der Mittelachse 13 in einem Abstand zueinander verteilt und in Achsenrichtung gesehen in einem Winkel zueinander versetzt. Dies verdeutlicht FIG. 3.

**FIG. 3** ist eine schematische Darstellung einer Anordnung des Abstandshalters 11 aus FIG. 2 in einem Katalysatorrohr (nicht dargestellt) mit Blickrichtung entlang der Mittelachse des Abstandshalters bzw. der Rohrachse des Katalysatorrohrs. Die Distanzhalter 15 sind in Umfangsrichtung winkelversetzt, sodass die Distanzhalter 15 in der Darstellung um 120° versetzt zueinander angeordnet sind. Die Distanzhalter 15 tragen an ihrem distalen Ende im Zentrum des Abstandshalters eine Buchse 17. In dieser Buchse 17 ist eine Hülse 18 geführt, in der der Temperaturmessfühler 19 angeordnet ist. Die Katalysatorschüttung 8 kann sich aufgrund der winkelversetzten Anordnung der Distanzhalter 15 auch innerhalb des zylindrischen Grundkörpers des Abstandshalters 11 verteilen.

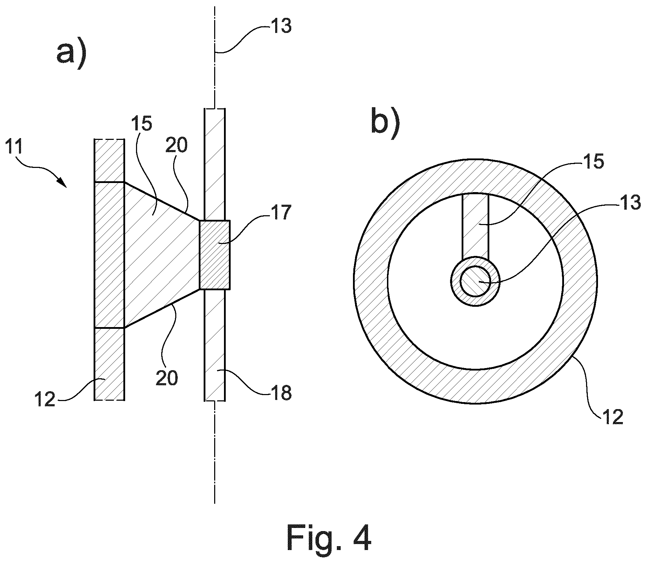

**FIG. 4** zeigt in einer Seitenansicht (Darstellung a) und in einer Draufsicht (Darstellung b) entlang der Mittelachse 13 Details einer Variante des Abstandshalters 11 aus FIG 2. Der Distanzhalter 15 weist in Achsenrichtung der Mittelachse 13 zwei voneinander abgewandte schräge Flächen 20 auf, sodass sich der Distanzhalter 15 zum distalen Ende hin, wo die Buchse 17 angeordnet ist, verjüngt. In der Buchse 17 ist die Hülse 18 geführt. In Darstellung b) ist zudem dargestellt, dass die durch den Distanzhalter 15 bereitgestellte Führung die Hülse mit dem Temperaturmessfühler in zentraler Position und damit zentral im Gasstrom im Katalysatorrohr positioniert.

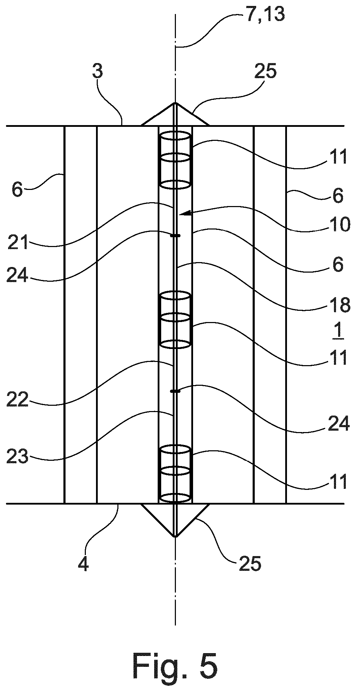

**FIG. 5** zeigt in einer schematischen Schnittansicht durch einen Reaktor 1, wie in FIG. 1, die Anordnung einer Temperaturmesseinrichtung 10 mittels der Haltanordnung in einem Katalysatorrohr 6. In der Hülse 18 ist der Temperaturmessfühler angeordnet. Die Hülse besteht aus drei Einzelsegmenten, den Hülsensegmenten 21, 22 und 23, die mittels Schraubverbindungen 24 miteinander gekoppelt sind. Jedes der Hülsensegmente 21, 22, 23 ist an einem Abstandshalter 11 fixiert. Das obere Ende der Hülse 18 ist an eine Arretierung 25 angeschlossen, die auf der oberen Rohrplatte 3 positioniert ist. Das untere Ende der Hülse 18 ist an eine Arretierung 25 angeschlossen, die unterhalb der unteren Rohrplatte 4 positioniert ist. Der Abstandshalter 11 kann so gestaltet sein, dass eine Rotation der Hülse möglich ist, aber eine relative Verschiebung in Achsenrichtung 7 bzw. 13 unterbunden wird.

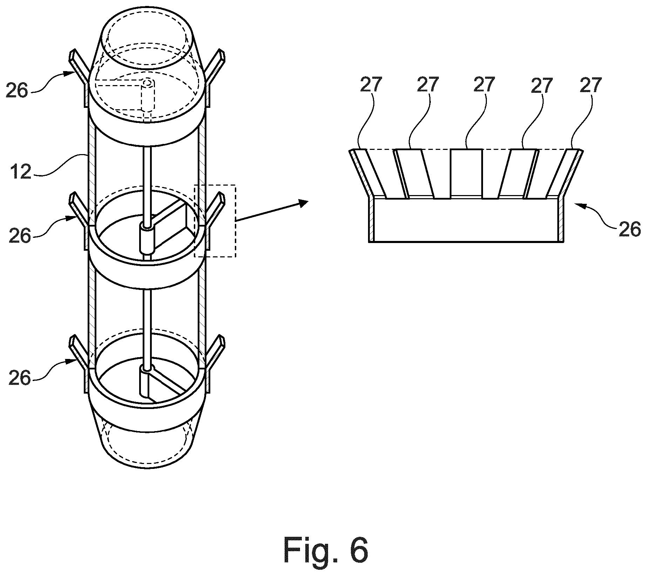

**FIG. 6** ist eine Darstellung einer Abwandlung des Abstandshalters aus FIG. 2. An den Außenseiten der zylindrischen Segmente des zylindrischen Grundkörpers 12 sind Randabweiser 26 angeordnet, die sich über den Umfang der zylindrischen Segmente erstrecken. Wie die Detaildarstellung des Randabweisers 26 zeigt, umfassen die Randabweiser eine Anzahl an Abweiserarmen 27, die nach außen, d.h., in Richtung Innenwand des Katalysatorrohrs, gerichtet sind.

### Bezugszeichenliste

- 1: Flüssigkeitsgekühlter Reaktor
- 2: Mantelraum
- 3: Obere Rohrplatte
- 4: Untere Rohrplatte
- 5: Reaktorwand
- 6: Katalysatorrohr
- 7: Rohrachse
- 8: Katalysatorschüttung
- 9: Flüssigkeitskühlung
- 10: Temperaturmesseinrichtung
- 11: Abstandshalter
- 12: Zylindrischer Grundkörper
- 13: Mittelachse
- 14: Aussparung
- 15: Distanzhalter
- 16: Konusfom
- 17: Buchse
- 18: Hülse
- 19: Temperaturmessfühler
- 20: Schräge Flächen
- 21: Hülsensegment
- 22: Hülsensegment
- 23: Hülsensegment
- 24: Verschraubung
- 25: Arretierung
- 26: Randabweiser
- 27: Abweiserarm

## Patentansprüche

1. Flüssigkeitsgekühlter Reaktor (1) zur exothermen katalytischen Umwandlung eines Reaktionsgases, insbesondere zur Synthese von Methanol, umfassend, einen Reaktorbehälter mit einem Mantelraum (2);
eine Vielzahl von in dem Mantelraum (2) vertikal angeordneten Katalysatorrohren (6), die sich entlang einer jeweiligen Rohrachse (7) erstrecken und die mit einer Katalysatorschüttung (8) befüllt sind,
eine Flüssigkeitskühlung (9), die dazu eingerichtet ist, dass die Katalysatorrohre (6) von einer Kühlflüssigkeit umströmt werden, um die bei der exothermen Reaktion entstehende Wärme abzuführen;
**dadurch gekennzeichnet, dass** in wenigstens einem der Katalysatorrohre (6) eine Temperaturmesseinrichtung (10) angeordnet ist, wobei die Temperaturmesseinrichtung (10) wenigstens einen Temperaturmessfühler (19) umfasst und dazu angepasst ist, die Temperatur in dem Katalysatorrohr (6) an mehreren Stellen entlang der Rohrachse (7) zu messen,
wobei eine Halteanordnung vorgesehen ist, die dazu eingerichtet ist, die axiale Position der Temperaturmesseinrichtung (10) in dem Katalysatorrohr (6) zu fixieren, wobei die Halteanordnung mehrere eines Abstandshalters (11) umfasst, die in dem Katalysatorrohr (6) entlang der Rohrachse (7) verteilt sind und die dazu eingerichtet sind, die Temperaturmesseinrichtung (10) in einem definieren Abstand zur Innenwand des Katalysatorrohres (6) zu positionieren.

2. Flüssigkeitsgekühlter Reaktor (1) nach Anspruch 1, wobei der Abstandshalter (11) einen zumindest abschnittsweise zylindrischen Grundkörper (12) umfasst, der an der Innenwand des Katalysatorrohres (6) positioniert ist und von dem sich ein oder mehrere Distanzhalter (15) zumindest teilweise radial nach innen erstrecken und eine Führung für die Temperaturmesseinrichtung (10) bereitstellen.

3. Flüssigkeitsgekühlter Reaktor (1) nach Anspruch 2, wobei der Abstandshalter (11) mehrere Distanzhalter (15) umfasst, die entlang der Rohrachse (7) in einem Abstand zueinander verteilt sind; vorzugsweise sind die Distanzhalter (15) in Achsenrichtung (7) des Katalysatorrohres (6) gesehen in einem Winkel zueinander versetzt, vorzugsweise gleichmäßig um die Rohrachse (7) verteilt.

4. Flüssigkeitsgekühlter Reaktor (1) nach einem der Ansprüche 2 bis 3, wobei der wenigstens eine Distanzhalter (15) in wenigstens einer Richtung entlang der Rohrachse (7) eine schräge oder abgerundete Fläche (20) aufweist; bevorzugt eine Schräge in einem Winkel von 40° bis 50°, besonders bevorzugt 45°, zur Rohrachse (7).

5. Flüssigkeitsgekühlter Reaktor (1) nach einem der Ansprüche 2 bis 4, wobei der zylindrische Grundkörper (12) ein oder mehrere Aussparungen (14) in einer umlaufen Seitenwand aufweist.

6. Flüssigkeitsgekühlter Reaktor (1) nach einem der vorhergehenden Ansprüche, wobei der Abstandshalter (11) ein oder mehrere Randabweiser (26) umfasst, wobei der Randabweiser (26) dazu angepasst ist, einen direkten Kontakt zwischen dem Außenumfang des Abstandshalters (11) und der Innenwand der Katalysatorrohres (6) zu verhindern.

7. Flüssigkeitsgekühlter Reaktor (1) nach einem der vorhergehenden Ansprüche, wobei die Halteanordnung Arretierungen (25) umfasst, die an einer oberen und unteren Rohrplatte (3, 4) festgelegt sind, zwischen denen sich die Katalysatorrohre (6) erstrecken.

8. Flüssigkeitsgekühlter Reaktor (1) nach einem der vorhergehenden Ansprüche, wobei die Temperaturmesseinrichtung (10) in einer Hülse (18) aufgenommen ist.

9. Flüssigkeitsgekühlter Reaktor (1) nach Anspruch 8, wobei die Hülse (18) mehrere Hülsensegmente (21, 22, 23) umfasst, die axial miteinander koppelbar sind; bevorzugt sind zumindest einige der Hülsensegmente (21, 22, 23) jeweils an einem Abstandshalter (11) angebracht, sodass das Hülsensegment (21, 22, 23) zusammen mit dem Abstandshalter (11) als Baugruppe mit wenigstens einem weiteren Hülsensegment (21, 22, 23) koppelbar ist.

10. Verfahren zur Methanolsynthese unter Verwendung eines Reaktors (1) nach einem der vorhergehenden Ansprüche, bei dem ein Synthesegas durch die Katalysatorrohre (6) geleitet und zumindest teilweise umgesetzt wird und eine Temperaturmessung an mehreren Stellen entlang der Rohrachse (7) eines oder mehrerer Katalysatorrohre (6) mittels einer in dem jeweiligen Katalysatorrohr (6) angeordneten Temperaturmesseinrichtung (10) vorgenommen wird; bevorzugt wird auf Grundlage einer vorgenommenen Temperaturmessung ein Temperaturprofil erstellt.
